Europäisches Patentamt

European Patent Office

Office européen des brevets

(19)

(11) **EP 1 173 710 B1**

(12) **EUROPÄISCHE PATENTSCHRIFT**

(45) Veröffentlichungstag und Bekanntmachung des Hinweises auf die Patenterteilung:
**31.03.2004 Patentblatt 2004/14**

(51) Int Cl.$^7$: **F23D 14/60**, G01N 11/08, G01N 33/22, F23N 1/02

(21) Anmeldenummer: **00938617.8**

(22) Anmeldetag: **26.04.2000**

(86) Internationale Anmeldenummer:
**PCT/EP2000/003761**

(87) Internationale Veröffentlichungsnummer:
**WO 2000/065280 (02.11.2000 Gazette 2000/44)**

(54) **VORRICHTUNG ZUR EINSTELLUNG DES OXIDATIONSMITTEL/BRENNSTOFFGEMISCHES IN DER ZULEITUNG EINES BRENNERS**

DEVICE FOR ADJUSTING THE OXIDATION AGENT/FUEL MIXTURE IN THE FEEDING PIPE OF A BURNER

INSTALLATION PERMETTANT DE MODIFIER LE MELANGE OXYDANT/COMBUSTIBLE DANS LA CONDUITE D'ALIMENTATION D'UN BRULEUR

(84) Benannte Vertragsstaaten:
**AT BE CH CY DE DK ES FI FR GB GR IE IT LI LU MC NL PT SE**

(30) Priorität: **26.04.1999 DE 19918901**

(43) Veröffentlichungstag der Anmeldung:
**23.01.2002 Patentblatt 2002/04**

(73) Patentinhaber:
• **Durst, Franz, Prof. Dr.**
  **91094 Langensendelbach (DE)**
• **Glass, Josef, Dipl.-Ing**
  **04155 Leipzig (DE)**
• **Pickenäcker, Karin, Dr.-Ing.**
  **68623 Lampertheim (DE)**
• **Trimis, Dimosthenis, Dr.-Ing.**
  **90762 Fürth (DE)**
• **Wawrzinek, Klemens, Dipl.-Ing.**
  **91054 Erlangen (DE)**

(72) Erfinder: **DURST, Franz**
  **D-91094 Langensendelbach (DE)**

(74) Vertreter: **Gassner, Wolfgang, Dr. et al**
  **Dr. Gassner & Partner Patentanwalt,**
  **Nägelsbachstrasse 49a**
  **91052 Erlangen (DE)**

(56) Entgegenhaltungen:
  EP-A- 0 628 815        GB-A- 1 571 906
  GB-A- 2 296 091        US-A- 4 384 792
  US-A- 5 311 447

**Beschreibung**

Die Erfindung betrifft eine Vorrichtung zur Einstellung des Oxidationsmittel/Brennstoffgemisches in der Zuleitung eines Brenners mit einer Einrichtung zur Änderung der Zusammensetzung des Oxidationsmittel/Brennstoffgemisches und einem Meßgerät zur Erfassung eines Zustandes des Oxidationsmittels/Brennstoffgemisches beim Verbrennen sowie einer Schaltung zur Steuerung der Einrichtung zur Änderung der Zusammensetzung in Abhängigkeit des durch das Meßgerät erfaßten Zustands. Eine solche Vorrichtung ist aus Dokument, H. Henrich und M. Hoppe "Signale aus der Flamme - Luftzahlerkennung aus dem Ionisationsstrom; Neue Möglich Keiten für die Kesseltechnik", Rahrgas Forum, Ausgabe 7, Essen 1998.

In der Heizungstechnik besteht seit langem die Problematik, daß das Verbrennungsverhalten von Gasbrennern wesentlich von der Qualität des örtlich vorliegenden Gases abhängig ist. Die Qualität wird durch den oberen Wobbeindex Wo charakterisiert, der gemäß Buderus Heiztechnik GmbH: Handbuch der Heizungstechnik, Beuth Verlag GmbH, Berlin, Wien, Zürich, 33. Auflage 1994, für Erdgas vom Typ L im Bereich von 10,5 bis 13 KWh/m$^3$ und für Erdgas vom Typ H im Bereich von 12,0 bis 15,7 KWh/m$^3$ liegt. Es wird angestrebt, Brenner zu bauen, die nicht nur mit Gasen der jeweils einen Gruppe sondern mit Gasen beider Gruppen betrieben werden können. Hochkalorische Gase haben einen hohen, niederkalorische einen geringen Oxidationsmittelbedarf. Wenn hier im folgenden Oxidationsmittel genannt ist, ist damit im allgemeinen Luft gemeint, wobei aber auch speziell Sauerstoff angereicherte Luft oder ganz andere Oxidationsmittel verwendet werden können.

Bei konventionellen Brenner-Regelungen wird die Gasmengenzufuhr nicht an die Qualität des Gases angepaßt, was zu einer Verschiebung der Luftüberschußzahl und somit im allgemeinen zu einer unerwünschten Veränderung der Verbrennungsprozesses führt. Bei extrem hochkalorischen Gasen kann das aber dazu führen, daß zu wenig Oxidationsmittel im Brenngasgemisch vorliegt und somit eine erhöhte Schadstoffproduktion entsteht, wohingegen die Flamme bei sehr niederkalorischen Gasen durch die zu große Oxidationsmittelmenge komplett ausgeblasen werden kann. Dies führt dann wiederum zu Einschränkungen des Betriebsbereichs eines Brenners, weil die gesetzlich vorgeschriebenen Emissionsgrenzwerte durch geringen Wirkungsgrad oder schlichtes Erlöschen der Flamme überschritten werden können. Ein Brenner sollte also derart einstellbar sein, daß alle möglichen Gasqualitäten bzw. Qualitätsklassen sicher und mit geringen Emissionen verbrannt werden können.

Die gleiche Problematik ist auch bei der Verbrennung von gasförmigen Brennstoffen, insbesondere Gasen der ersten bis vierten Gasfamilie, Alkanen, Alkenen, Alkinen sowie Gasmischungen, die in hohem Maße Methan, höhere Kohlenwasserstoffe, Wasserstoff, Stickstoff, Kohlenmonoxid, Kohlendioxid und Luft beinhalten, gegeben. Auch hier sollte die Brennstoff bzw. Luft- oder Oxidationsmittelmenge an die Brennstoffart angepaßt werden können.

Das gleiche gilt für flüssige oder mehrphasige Brennstoffe. Zu flüssigen Brennstoffen zählen beispielsweise Treibstoffe, verflüssigte Brenngase und Öle, insbesondere Diesel-, Heiz- und Rapsöle. Zu den mehrphasigen zählen beispielsweise in Gasphase transportierte feste Brennstoffpartikel, siedende Brennstoffe oder Inertgase enthaltende flüssige Brennstoffe.

Es wird mehr und mehr eine Technik eingesetzt, mit der die Qualität des Brennstoffs oder allgemein der Zustand des Brennstoffs erkannt und die Luftzahl entsprechend gesteuert wird. Dies schließt natürlich nicht aus, daß man die Qualität des Brennstoffs auch durch Zusätze entsprechend einem konstanten Heizwert einstellen kann.

Im allgemeinen wird die Zusammensetzung für den Brenner so gesteuert, daß der Brenner wenigstens in der Nähe eines optimalen Betriebspunkts arbeitet. Dadurch wird der Betriebsbereich eines Brenners erweitert, wobei allerdings komplexe Verfahren zur genauen Regelung der benötigten Luft oder Oxidationsmittelmenge, die dann vor allem auf Messungen im Brennerbetrieb basieren, technisch nicht realisiert werden können, wenn der Aufwand für einen Brenner möglich gering gehalten werden soll.

Im folgenden seien einige Konzepte genannt, um eine von der Brenngaszusammensetzung unabhängige Verbrennung zu realisieren. Eine Möglichkeit beruht auf der Messung des Ionisationsstroms bei der Verbrennung. Dazu sei auf die Artikel M. Herrs, R. Merker, Dr. R. Naumann, H. Nolte: "Optimierung von Verbrennungsprozessen mittels eines Ionisations-Flammen-Managements," Gas Wärme International, 47 (1998), Heft 2; und "Signale aus der Flamme, Luftzahlerkennung aus dem Ionisationsstrom: Neue Möglichkeiten für die Kesseltechnik," Ruhrgas Forum, Ausgabe 7, Essen, 1998; verwiesen.

Die Ionisationselektroden sind dabei Bestandteil der Sicherheitskette des Brenners, die bei Verlöschen der Flamme die Gaszufuhr abschaltet. Das eingesetzte Meßverfahren beruht dabei auf dem physikalischen Effekt, daß sich die Moleküle des Gas-Luft-Gemisches und seine Reaktionsprodukte bei den hohen Temperaturen in der Flamme in elektrisch leitfähige Ionen aufspalten. Wird zwischen Brenner und Ionisationselektrode eine Spannung angelegt, fließt ein elektrischer Strom solange die Flamme brennt.

Neben der Erkennung des Flammen-Ein/Aus-Zustands enthält der Ionisationsstrom aber noch weitere Informationen über den Verbrennungszustand, unter anderem über die Verbrennungstemperatur und damit indirekt über die Luftzahl. Durch Kalibrierung wird eine Referenz bezüglich eines stöchiometrischen Betriebs hergestellt, der durch den Maximalwert des Ionisationsstroms gekennzeichnet ist. Der überstöchiometrische Arbeitspunkt wird relativ zu diesem Referenzwert festgelegt und bildet die Basis einer Regelung.

Eine andere Möglichkeit zum Erreichen verbesserter Verbrennungsbedingungen beruht auf einer Messung der Oxidationsmittelkonzentration im Abgas, wie dies beispielsweise auch in der Automobiltechnik realisiert wird. Dabei kommen sogenannte Lambdasonden, wie sie beispielsweise in der Produktinformation der Firma Gasmodul (Honeywell-Co.): "Kesselsteuerung MCBA 1400 und GMS-10 $O_2$-Sensor" dargestellt sind, zum Einsatz. Diese Lambdasonden bestehen im wesentlichen aus zwei Elektroden, die durch einen Feststoffelektrolyt miteinander verbunden sind. Dieser Feststoffektrolyt ist in der Lage Ionen des hier als Oxidationsmittel eingesetzten Sauerstoffs zu leiten. Bei Anlegen einer elektrischen Gleichspannung entsteht eine elektrochemische Pumpwirkung, aufgrund der Sauerstoffionen durch den Feststoffelektrolyt geleitet werden. Durch das selektive Pumpen der Sauerstoffionen entsteht ein Konzentrationsgefälle, das eine Nernst-Spannung erzeugt. Die Zeitspanne, die benötigt wird, damit die Spannungsdifferenz einen Sollwert erreicht, wird als Maß für den partiellen Oxidationsmitteldruck der Umgebung verwendet.

Ein weiteres Verfahren zur Regelung eines Gasbrenners unter Berücksichtigung der Gasbeschaffenheit ist die Nutzung der Lichtleitmeßtechnik. Die von der Flamme emittierte Strahlung, die von der Gemischzusammensetzung abhängig ist, wird von einer optischen Sonde erfaßt und über einen Lichtleiter zu einem Photomultiplier geführt. Das Signal des Photomultipliers kann dann als Regelgröße für die Gasmengenzufuhr verwendet werden. Ein derartiges Regelsystem ist beispielsweise in dem Artikel "Neues Regelsystem für Vormischbrenner, Lichtleiter-Meßtechnik erfaßt Flammensignale", Ruhrgas Forum, Ausgabe 4, Essen, Dezember 1992, angegeben.

Zur Messung und Regelung der Wärmenmengenzufuhr existiert ein weiteres Verfahren gemäß der deutschen Offenlegungsschrift DE 43 36 174 A1, bei der durch Messung des Volumenstromes des Gases, des Drucks, der Temperatur, der Dichte und der Schallgeschwindigkeit unter Normalbedingungen sowie der Schallgeschwindigkeit unter Betriebsbedingungen auf die im Brennstoff transportierte Wärmemenge geschlossen wird.

Gemäß der DE 29 28 739 wird ein Teilstrom des Brenngases abgezweigt und in einem Kaloriemetergefäß umgesetzt. Aus der frei werdenden Wärmemenge kann mittels eines funktionellen Zusammenhangs auf die Brenngaszusammensetzung rückgeschlossen werden. Der so gelieferte Meßwert kann als Signal für Regelvorgänge verwendet werden, um den Wobbeindex des Gasstroms durch Zuführung eines Gases konstant zu halten oder den Gasmengenstrom in zweckmäßiger Weise zu verändern. Derartige Verfahren werden von Gasversorgern zur Überprüfung bzw. Steuerung der Gasqualität bzw. in größeren Industrieanlagen zur Brennereinstellung verwendet.

Im Prinzip beinhalten alle diese Verfahren die Messung verschiedener physikalischer Größen über ein Meßgerät und eine Regelung und Steuerung über eine Schaltung, mit der eine Einrichtung zur Änderung der Zusammensetzung gemäß der vom Meßgerät gelieferten Signale das Mischen steuert bzw. regelt. Dabei wird eine Steuerung gemäß der Zielsetzung durchgerührt, einen Verbrennungsprozeß an Schwankungen der Brennstoffqualität anzupassen.

Bisher haben sich technisch nur derartige Verfahren für Brenner, insbesondere Haushaltsbrenner, durchgesetzt, die über Ionisationsstrommessung oder eine Lambdasonde zur Steuerung während oder nach der Verbrennung gewonnene Meßwerte ermitteln. Damit ist es allerdings nicht möglich, bereits vor dem Start auf die Änderung der Gasqualität zu reagieren.

Als weitere entscheidende technischen Nachteile sind bezüglich der Lambdasonde geringe Lebensdauer, bzgl. der Ionisationsstrommessung die auftretende thermische Belastungen des Brennersystems mit der Folge einer hohen Schadstoffentwicklung beim Anfahren der Stöchiometriepunktes zu nennen.

Bei der Ionisationsstrommessung können ferner Fehlmessungen auftreten, weil prinzipiell für jede neue Leistungseinstellung eine neue Kalibrierung notwendig wäre. Eine kontinuierliche Modulation ist deshalb beispielsweise technisch außerordentlich schwer zu realisieren, man beschränkt sich auf den Betrieb mit einer begrenzten Anzahl von Leistungsstufen.

Die vorher genannte kalorimetrische Bestimmung des Gases bedeutet einen gewissen Energieverlust. Aus Kostengründen ist eine kalorimetrische Bestimmung der Gaszusammensetzung für Heizungsanlagen nicht empfehlenswert.

Aufgabe der Erfindung ist es, eine verbesserte Vorrichtung zur Einstellung des Oxidationsmittel Brennstoffverhältnisses in der Zuleitung eines Brenners der eingangs genannten Art zu schaffen, deren Aufwand in vertretbaren Grenzen bleibt.

Die Aufgabe wird mit Hilfe einer Vorrichtung gelöst, bei der das Meßgerät den Zustand von mindestens einem Teilgemisch des Oxidationsmittel/Brennstoffgemisches über die Viskosität oder eine Funktion derselben vor dem Verbrennen erfaßt und bei der dieses Meßgerät in Strömungsrichtung vor der Einrichtung zur Änderung der Zusammensetzung angeordnet ist und die Zusammensetzung des Oxidationsmittel/Brennstoffgemisches gemäß einer Funktion der erfaßten Viskosität mittels der Schaltung zur Steuerung der Einrichtung steuert oder bei der es hinter der Einrichtung zur Änderung der Zusammensetzung angeordnet ist und mittels der Schaltung zur Steuerung der Einrichtung die Zusammensetzung des Oxidationsmittel/Brennstoffgemisches regelt.

Daß mit einer derartigen Vorrichtung die Aufgabe vorteilhaft gelöst wird, ist zunächst unerwartet. Man hätte erwartet, daß der Fachmann die bekannten Verfahren mittels Ionisationsstrommessungen oder Lambdasonde durch spezielle Konstruktion bzw. Ermitteln weiterer Parameter zur Verbesserung der Steuerung optimiert hätte. Hier wird jedoch ein ganz anderer Weg beschritten, insbesondere schon dadurch, daß noch vor dem Brenner eine Messung der Zusammensetzung erfolgt. Das einzige diesbezüglich bekannte Verfahren wäre die kalorimetrische Bestimmung des Heizwertes gewesen, die aber immer einen gewissen Energieverlust mit sich bringt und bisher auch nicht zur Brennersteuerung eingesetzt wurde.

Weiter wird erstmalig die Viskosität von zumindest einem Teilgemisch ermittelt, eine physikalische Größe, die bisher bei der Brennersteuerung nicht berücksichtigt wurde, möglicherweise weil der später noch eingehender erläuterte Zusammenhang zwischen Viskosität und Heizwert in Fachkreisen wenig bekannt ist.

Nach Fertigstellung der Erfindung wurde festgestellt, daß sie neben der Lösung der vorstehend genannten Aufgabe noch weitere Vorteile bietet. So kann die erfindungsgemäße Vorrichtung bereits vor der Zündung der Flamme für eine Anpassung des Brenners an veränderte Eigenschaften des Brennstoffes eingesetzt werden. Dadurch läßt sich der Zündprozeß erleichtern. Insbesondere bei niederkalorischen Gasen auftretende Zündschwierigkeiten können üblicherweise nur mit aufwendigen Optimierungsmaßnahmen der Brenneranordnungen behoben werden. Des weiteren kann hiermit die Anfahremissionen gezielt auf ein niedriges Niveau gesteuert werden.

Ferner ist ein kontinuierlicher Betrieb möglich, da die Luftmenge selbst bei Drosselung der Brennstoffzufuhr in geeigneter Weise nachgeführt wird. Ein Brenner, der mit einer derartigen Vorrichtung betrieben wird, muß daher zum Heizen auf verschiedenen Stufen nicht ein- und ausgeschaltet werden. D.h. Zündprozesse, die bei herkömmlichen Brennen wesentlich zur Abgasemission eines Brenners beitragen, können vermieden werden, was ebenfalls zu einem verbesserten Umweltschutz beiträgt.

Insgesamt ist jedoch herauszustellen, daß es mit Hilfe dieser Technik möglich wird, den Brennstoff vollständiger zu verbrennen, was sich auch bei den Kosten eines Brenners im Betrieb günstig auswirkt. Aufgrund der dadurch nahezu vollständigen Verbrennung können auch Wartungszyklen, beispielsweise zum Beseitigen von Brennstoffresten, reduziert werden.

Aufgrund des bei der Vorrichtung völlig neu eingesetzten Verfahrens läßt sich diese sogar zur Brennersteuerung für die verschiedensten Arten von Brennstoff einsetzen. Weiter kann die Vorrichtung, je nach Anordnung des Meßgerätes, entweder vor oder nach der Einrichtung zur Änderung der Zusammensetzung, als Steuerung oder als Regelung eingesetzt werden. Eine Steuerung wird allerdings bevorzugt, weil sich herausgestellt hat, daß sich der Wobbeindex bei einer Erfassung der Viskosität an einem reinem Brennstoffgemisch ohne zugeführtes Oxidationsmittel wesentlich besser bestimmen läßt.

Zur Erläuterung der Erfindung bezüglich des physikalischen Sachverhalts wird auch auf die DE 29 28 739 und die US-A-4 384 792 verwiesen, bei der die Erkenntnis, daß die Brennstoffeigenschaften von Erdgasen verschiedener Herkunft und Zusammensetzung mit großer Genauigkeit als Funktion der dynamischen Viskosität der Fluide bestimmt werden können, veröffentlicht wurde. Diese Erkenntnis wurde bisher bei der Steuerung von Brennern nicht verwertet. In der Druckschrift wird dieses Verfahren einzig dafür empfohlen, ein vernünftiges Maß für die Abrechnung gelieferter Brennstoffmengen anzugeben. Das Verfahren hat sich aber auch dort nicht durchgesetzt; auch zur Abrechnung wird im wesentlichen weiter mit kalorimetrischen Methoden gearbeitet.

Die Grundlage für die Ermittlung des Heizwerts bzw. des Wobbeindex aus der Viskosität sei hier kurz erläutert:

Der obere Wobbeindex $W_o$ bei dem vorliegenden Druck- und Temperaturzustand des Brenngases ist bekanntlich eine wichtige Brennstoffqualitätsgröße von gasförmigen Brennstoffen und wird definiert durch den Ausdruck:

$$W_o = \frac{H_o}{\sqrt{\dfrac{\rho}{\rho_{L,n}}}}, \tag{1}$$

wobei $H_o$ den Betriebsbrennwert, $\rho$ die Dichte des Brenngases und $\rho_{L,n}$ die Normdichte (273,15 K und 1013,25 hPa) der Luft bezeichnen. Analog wird auch der untere Wobbeindex $W_u$ definiert, wobei statt des Brennwerts $H_o$ der Heizwert $H_u$ herangezogen werden muß.

Die Viskosität kann beispielsweise bestimmt werden, wenn unter der Voraussetzung einer laminaren Gasströmung in einer Kapillare oder einem Rohr folgende physikalische Gesetzmäßigkeit als Grundlage dient (Hagen-Poiseulle-Gesetz):

$$\eta = \frac{\Delta\rho \cdot \pi \cdot R^4}{8 \cdot \ell \cdot V} \tag{2}$$

Dabei bedeuten:

η    die dynamische Gasviskosität,

$\Delta p$    die Druckdifferenz zwischen Ein- und Austritt bzw. den zwei Druckmeßstellen,

R    der Radius der Kapillare,

$\ell$    die Länge der Kapillare und

$\dot{V}$    den Brenngasvolumenstrom.

Als Alternative zu der im laminaren Bereich gültigen Gleichung (2) wäre auch die Ausnutzung des etwas komplexeren rohrrauigkeitsabhängigen Zusammenhangs für nicht vollständig ausgebildete Turbulenz, d.h. im Übergangsgebiet zwischen laminarer und vollturbulenter Strömung möglich. Eine weitere Alternative besteht in der Verwendung von nicht kreisförmigen Rohren, insbesondere rechteckigen, kreisringförmigen oder ovalen Querschnitts, wobei dann aber andere von Gleichung (2) abweichende funktionale Zusammenhänge angesetzt werden müssen. Auch für mehrphasige Fluide kann ein Zusammenhang zwischen effektiver Viskosität und Druckabfall, der sich komplexer als Gleichung (2) darstellt, genutzt werden.

Für eine Bestimmung der dynamischen Viskosität η müssen also außer den geometrischen Daten noch $\dot{V}$ und $\Delta p$ bekannt sein. Für eine Brennersteuerung sollte allerdings der Volumenstrom gering sein, um einerseits den Platzbedarf für die Vorrichtung durch die Nutzung kleiner Kapillaren gering zu halten und anderseits bei großen Volumenströmen signifikanter werdende Einlaufeffekte der Strömung, die zu größeren Meßfehlern rühren können, gering zu halten. Aus der Nutzung kleiner Kapillaren resultieren darüber hinaus geringe Kosten. Die Messung sehr kleiner Volumenströme ist allerdings sehr schwierig. Deshalb sollte möglichst, ein konstanter, sehr geringer Volumenstrom bereitgestellt werden. Dies kann insbesondere gemäß einer Weiterbildung der Erfindung mittels einer Mikropumpe, insbesondere eine Halbleitermikropumpe, geschehen. Der Druckverlust kann dann auf übliche Weise (z. B. piezoelektrisch) gemessen werden. Für die Ermittlung der dynamischen Viskosität η kann außerdem die Temperaturabhängigkeit der dynamischen Viskosität berücksichtigt werden. Die Funktionsweise eines bei der Erfindung eingesetzten, vorteilhaft ausgestatteten Viskositätssensors kann folgendermaßen beschrieben werden:

Die Pumpe fördert einen konstanten bekannten Volumenstrom durch die Kapillare, über welcher der Druckabfall gemessen wird. Unter Berücksichtigung der Gastemperatur wird dann ein elektronisches Signal bestimmt, das der Viskosität und damit auch dem Wobbeindex oder anderen Brennstoffqualitätsgrößen, insbesondere minimaler Luftbedarf, Brennwert und Heizwert, entspricht. Als Alternativen sind auch eine Messung von $\dot{V}$ und $\Delta p$ oder eine kontrollierte Einstellung von $\Delta p$ und eine Messung von $\dot{V}$ durchführbar. Auf die Messung oder Vorgabe eines besonderen Drucksignals kann sogar verzichtet werden, wenn die Verlustleistungen der Dosierpumpen künftig derart gering sind, daß der Druckverlust über die Leistungsaufnahme der Pumpe bestimmt werden kann.

Die Erfindung nutzt die theoretische Überlegung, daß bei der Änderung der Gaszusammensetzung gilt:

$$\lambda_2 = \lambda_1 \cdot \sqrt{\frac{\rho_2}{\rho_1}} \cdot \frac{L_{\min,1}}{L_{\min,2}} \cong \lambda_1 \cdot \frac{W_{o,1}}{W_{o,2}} \cong \lambda_1 \cdot \frac{W_{u,1}}{W_{u,2}}, \tag{3}$$

wenn sich der Brennstoffvolumenstrom durch eine turbulente Drosselung umgekehrt proportional zur Wurzel der Dichte des Brennstoffs verhalten würde. Die Luftzahl $\lambda_2$ ändert sich allgemein in etwa proportional zum Verhältnis der Wobbeindizes ($W_o$ =oberer Wobbeindex; $W_u$ =unterer Wobbeindex) und der Primärluftzahl im Nennbetrieb $\lambda_1$. Dabei bedeuten:

$\rho_{1,2}$    die Brenngasdichte und

$L_{\min 1,2}$    den minimaler Luftbedarf

Erfolgt dagegen keine turbulente Drosselung, sondern eine laminare Durchströmung bei konstanten $\Delta p$, so gilt:

$$\lambda_2 = \lambda_1 \cdot \frac{\mu_2}{\mu_1} \cdot \frac{L_{\min,1}}{L_{\min,2}} \tag{4}$$

Wird ein Verfahren eingesetzt, bei dem sich der Brenngasvolumenstrom unabhängig von der Brenngasdichte verhalten würde, so gilt der einfache Zusammenhang:

$$\lambda_2 = \lambda_1 \cdot \frac{L_{\min,1}}{L_{\min,2}} \tag{5}$$

Die oben genannten Rechnungen zeigen, daß es im Idealfall für eine Bestimmung der Viskosität ausreicht, den Heizwert bzw. die notwendige Luftmenge zu bestimmen. Allerdings weichen die in der Praxis vorhandenen Bedingungen prinzipiell von den oben betrachteten idealisierten Bedingungen ab, so daß im allgemeinen kein einfacher Zusammenhang erwartet wird, und man sich eher auf eine gemessene Kennlinie verlassen sollte.

Diese Ermittlung des Heizwertes und damit des Wobbeindexes läßt sich jedoch noch gemäß einer vorteilhaften Weiterbildung der Erfindung verbessern, bei der mindestens eine Meßstelle zum Erfassen der Temperatur zumindest von einer Teilmenge des Oxidationsmittel/Brennstoffgemisches vorgesehen ist. Aufgrund der Temperaturmessung läßt sich die Viskosität und damit der Wobbeindex noch wesentlich besser erfassen. Die gemessene Temperatur kann aber auch als zusätzlicher Parameter eingeführt werden, um über eine Kennlinienschar den Wobbeindex bzw. den Heizwert und damit die benötigte Luft besser zu bestimmen.

Die Meßgenauigkeit wird vor allem gemäß einer vorteilhaften Weiterbildung der Erfindung erhöht, bei der das Meßgerät die Viskosität ausschließlich des Brennstoffes oder einer Funktion derselben erfaßt.

Würde dagegen die Viskosität des Luft/Brennstoffgemsiches ermittelt, müßten beispielsweise auch die Parameter der zugemischten Luft zur Einstellung verwendet werden, was die Vorrichtung unnötig komplizieren würde. Deswegen ist, wie oben schon ausgeführt, auch eine Steuerung günstiger als eine Regelung mittels der Vorrichtung.

Neben anderen bekannten Geräten zur Bestimmung der Viskosität ist das Meßgerät, wie es gemäß einer bevorzugten Weiterbildung der Erfindung vorgesehen ist, ein Rohrviskosimeter, mit dem die dynamische Viskosität über den Zusammenhang zwischen Druckabfall über ein Rohr, den Volumenstrom und gegebenenfalls auch die Temperatur ermittelbar ist. Ein derartiges Meßgerät weist insbesondere den Vorteil einer besonders einfachen und wenig aufwendigen Viskositätsbestimmung auf.

Bei einer anderen vorzugsweisen Weiterbildung der Erfindung ist vorgesehen, daß die Abmessungen des Rohrs so gewählt sind, daß die Strömung im wesentlichen laminar ist oder wenigsten im Übergangsbereich zwischen turbulenter und laminarer Strömung liegt.

Man beschränkt sich bei dieser Weiterbildung auf im wesentlichen laminare Strömungen. Dies hat den Vorteil einer sehr einfachen Bestimmung der Regelung gemäß den vorher angegebenen Gleichungen. Dagegen wäre es bei im wesentlichen turbulenten Strömungen empfehlenswert, für jede Vorrichtung zur besseren Bestimmung der Viskosität eine eigene Kennlinienschar zu bestimmen. Dies würde den Herstellungsprozeß und Aufwand zur Fertigung der erfindungsgemäßen Vorrichtung erhöhen.

Prinzipiell läßt sich bei derartigen Bedingungen jedes beliebige Rohr, ob gerade, ob gekrümmt oder aber auch in beliebigen Formen gebogen verwenden. Auch der Querschnitt kann prinzipiell beliebig gewählt werden. Insbesondere wird der Aufwand aber besonders verringert, wenn gemäß zweier bevorzugter Weiterbildungen der Erfindung erstens das Rohr gerade ist und zweitens das Rohr einen gleichmäßigen, insbesondere kreisförmigen Querschnitt, über seine Länge aufweist.

Dabei kann auch der Rohrdurchmesser prinzipiell beliebig gewählt werden. Es ist aber vor allen Dingen vorteilhaft, wenn nur wenig Gas für die Messung verwendet wird und der Druckanstieg für die Viskositätsbestimmung möglichst hoch wird, man also praktisch zu einer Kapillaren übergeht. Dies ist bei einer bevorzugten Weiterbildung der Erfindung berücksichtigt, bei der vorgesehen ist, daß der Rohrdurchmesser kleiner als 0,2 mm ist.

Diese Dimensionierung bedeutet vor allem auch in Kombination mit vorhergehenden Weiterbildungen der Erfindung eine besonders gute Erfassung der Viskosität über den Druckabfall oder die Strömungsgeschwindigkeit.

Mit Hilfe eines Viskosimeters läßt sich nämlich die Viskosität insbesondere durch Messung des Druckverlustes nach Durchströmen des Rohres oder auch durch die Strömungsgeschwindigkeit bei konstantem Druckgefälle bestimmen. Weiter ist es möglich, jede beliebige Kombination von Strömungsbedingungen und Druckabfall über die bekannten Strömungsgleichungen zur Bestimmung der Viskosität heranzuziehen.

Insbesondere ist aber gemäß einer Weiterbildung der Erfindung bevorzugt, daß der Druckabfall über das Rohr oder zumindest innerhalb eines Teilrohrstücks des Rohrs meßtechnisch erfaßbar ist, wobei dann die Strömungsgeschwindigkeit im wesentlichen konstant gehalten wird.

Druckänderungen am Eingang des Rohres können die Messung allerdings geringfügig verfälschen. Um derartigen störenden Effekten am Rohreingang möglichst entgegenzuwirken ist gemäß einer vorteilhaften Weiterbildung vorgesehen, daß der Rohreinlauf gerundet ist.

Insbesondere hat sich bezüglich der Festlegung der Rohrabmessungen eine Dimensionierung bezüglich einer Reynoldszahl keiner als 1000 als besonders vorteilhaft herausgestellt.

Meßtechnisch ist es besonders einfach, wenn eine steuerbare Pumpe zur Einstellung des Volumenstroms in dem Rohr vorgesehen ist. Bei konstant gehaltenem Volumenstrom kann die Viskositätsbestimmung einfach über eine Druckmessung und bei gegebenenfalls stark veränderlichen Temperaturen auch durch die Berücksichtigung der Temperatur über eine zusätzliche Temperaturmessung bestimmt werden. Dies kann vor allen Dingen dann leicht realisiert werden, wenn die Pumpe so gesteuert ist, daß der Volumenstrom gemäß einer vorzugsweisen Weiterbildung der Erfindung insbesondere über diese steuerbare Pumpe konstant geregelt wird.

Eine weitere Möglichkeit besteht aber bei der steuerbaren Pumpe darin, daß die Pumpe bezüglich eines konstanten Druckabfalls über dem Rohr geregelt wird. Dann erfolgt die Viskositätsbestimmung im wesentlichen über den Volumenstrom, der dann allein über die Pumpensteuerung bestimmbar ist. Bei entsprechender geforderter Meßgenauigkeit kann der Meßwert auch über eine zusätzlich gemessene Temperatur verbessert werden. Als Pumpen sind insbesondere Dosierpumpen, rotierende oder oszillierende Verdrängerpumpen, sowie rotierende oder oszillierende Verdrängungsverdichter einsetzbar. Gemäß einer bevorzugten Weiterbildung der Erfindung ist aber vorgesehen, daß die Pumpe eine Mikropumpe ist. Derartige Pumpen sind mikromechanische Bauelemente, die kostengünstig und einfach aufgebaut sind. Insbesondere sind sie aufgrund der geförderten kleinen Menge auch blasentolerant. Eine derartige Pumpe ist beispielsweise in dem Artikel " A self-priming and bubble-tolerant piezoelectric silicon micropump for liquids and gases", Micro Electro Mechanical Systems MEMS, 25.-29. Januar 1999, Deutschland von R. Linnemann und anderen beschrieben.

Besonders kompakt und wenig aufwendig wird die Vorrichtung gemäß zweier Weiterbildungen der Erfindung bei der die Halbleitermikropumpe sowie mindestens Teile der Schaltung (28), insbesondere auch der Druck- und Temperatursensor, auf einen gemeinsamen Substrat integriert sind und/oder der Druckabfall über ein Rohr über die Leistungsaufnahme der Pumpe bestimmbar ist.

Insbesondere bei der letzten Weiterbildung der Erfindung läßt sich ein Drucksensor sparen, indem man als Maß für den Druck die Leistungsaufnahme der Pumpe verwendet.

Verlustleistungen der Pumpen, insbesondere Mikropumpen sind heute so reproduzierbar, daß die Leistung über eine Kalibrierkurve einfach in den Druck oder sogar die gewünschte Viskosität kalibriert werden kann.

Bei einer anderen bevorzugten Weiterbildung der Erfindung ist eine Brennersteuerung vorgesehen, durch welche die Einstellung des Oxidationsmittel/Brennstoffgemisches schon vor der Zündung aktiv ist und durch welche gegebenenfalls die Zusammensetzung des Oxidationsmittel/Brennstoffgemisches während des Brennens kontinuierlich steuerbar ist. Damit werden die oben schon näher beschriebenen Vorteile eines kontinuierlichen Brennerbetriebs und insbesondere auch einer schadstoffarmen Zündung auf einfache Weise verwirklicht.

Bei besonders konstanten Bedingungen, beispielsweise bei Garantiewerten für Schwankungen des Wobbeindex durch die Gasversorgungsunternehmen, reicht es, wenn die Brennersteuerung vor und /oder auch während des Zündungsvorganges aktiv ist. Eine weitere Steuerung im kontinuierlichen Betrieb kann dann entfallen.

Andererseits ist es besonders bei sehr stark schwankenden Gasversorgungen empfehlenswert, die Steuerung auch nach der Zündung kontinuierlich oder in gleichmäßigen Meßintervallen zu betreiben. Der kontinuierliche Betrieb ist dabei vorzuziehen.

Insbesondere ist es vorteilhaft, wenn die Vorrichtung miniaturisiert ist, sie als Teil der für den Brenner verwendeten Gaseinheit auszulegen. Dieser Vorteil ergibt sich im Besonderen, wenn die Vorrichtung, wie oben schon angedeutet, als integriertes Halbleiterbauelement ausgeführt ist.

Der vermessene Brennstoff bzw. das vermessene Brennstoffgemisch kann natürlich im einfachsten Fall in die Umgebung abgeführt werden. Dies würde jedoch die Umwelt unnötig belasten, selbst wenn Mikropumpen mit nur kleinen Volumenströmen verwendet werden.

Um diese Umweltbelastung möglichst gering zu halten, wird die Erfindung bevorzugt dadurch weitergebildet, daß eine Einrichtung, insbesondere das Gebläse des Brenners, zur Rückführung des Teilgemisches in die Zuleitung des Brenners vorgesehen ist, die den Ausgangsdruck des gemessenen Teilgemisches unbeeinflußt läßt.

Wie dabei deutlich wird, ist dafür insbesondere das Gebläse besonders geeignet. Hierdurch wird in einfacher Weise ermöglicht, daß der Ausgangsdruck des Teilgemisches wesentlich unverändert bleibt, so daß auch meßtechnische Ungenauigkeiten in einfacher Weise vermieden werden können.

Bei einer vorzugsweisen Weiterbildung ist vorgesehen, daß dem Meßgerät ein Referenzgas zuführbar ist. Mit Hilfe dieses Referenzgases kann der Viskositätssensor kalibriert werden.

Weitere Vorteile und Besonderheiten der Erfindung ergeben sich auch aus der folgenden Darstellung von Ausführungsbeispielen unter Bezugnahme auf die begleitende Zeichnung. Es zeigen:

Fig. 1 bis 3    verschiedene Diagramme zur Darstellung des Zusammenhangs zwischen dynamischer Viskosität und unterschiedlichen, das Brennverhalten charakterisierenden Brenngaseigenschaften;

Fig. 4    einen möglichen Aufbau für ein Meßgerät zur Bestimmung der Viskosität;

Fig. 5    ein Ausführungsbeispiel für den Einsatz eines Meßgeräts gemäß Figur 4 in den Gesamtverbund einer Brennersteuerung;

Fig. 6    ein anderes Ausführungsbeispiel für den Einsatz eines Meßgeräts gemäß Figur 4 in den Gesamtverbund einer Brennersteuerung.

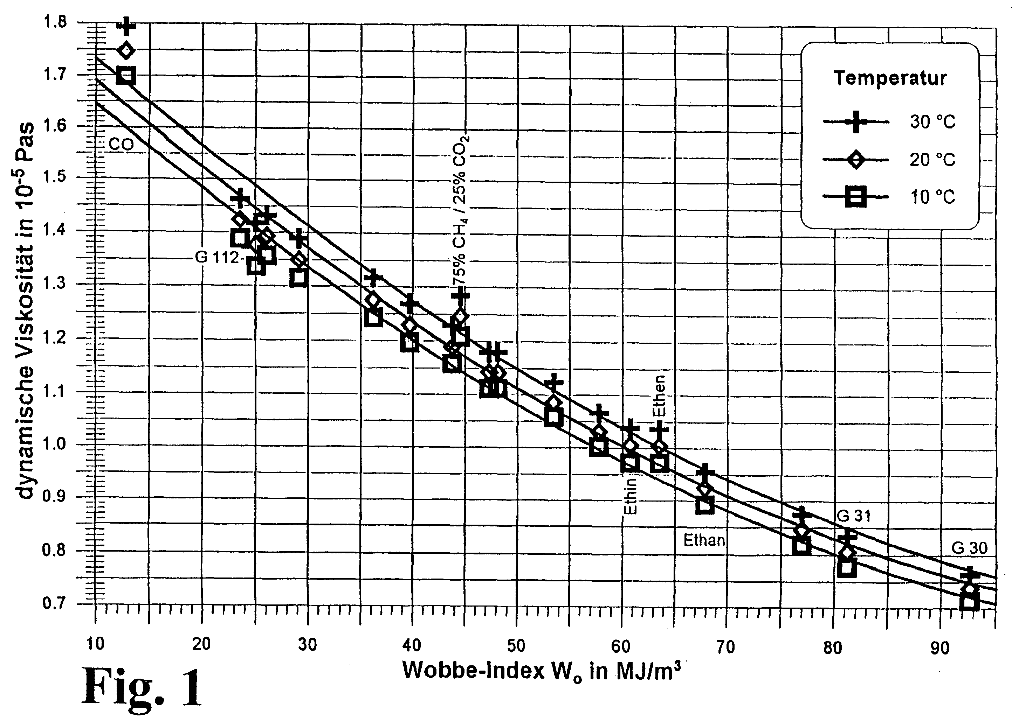

Ein wesentlicher physikalischer Begriff für die hier interessierenden Zusammenhänge ist der obere Wobbeindex, der als Brennwert durch Wurzel des Verhältnisses der Gasdichte zur Luftdichte unter Normalbedingungen definiert ist. Wie in Fig. 1 dargestellt wurde, existiert ein empirischer funktioneller Zusammenhang zwischen diesem Wobbeindex und der dynamischen Viskosität.

Der Zusammenhang zwischen der dynamischen Viskosität und dem Wobbeindex ist in Fig. 1 zu erkennen, bei der für verschiedene Brennstoffe einzelne Meßpunkte für diese beiden Größen eingetragen sind. Exemplarisch sind einige Brennstoffe mit der für die Heizungstechnik in der Europäischen Gemeinschaft branchenüblichen Bezeichnung benannt.

Demnach ergibt sich eine einfache funktionelle Abhängigkeit zwischen der dynamischen Viskosität und dem Wobbeindex, der mittels der durchgezogener Linien dargestellt ist, und der im Bereich von ± 10 % bei unterschiedlichen Brennstoffen im wesentlichen eingehalten wird.

In Fig. 1 sind vor allen Dingen auch Daten für verschiedene Temperaturen des Brennstoffes bei 10°C, 20 °C und 30 °C aufgetragen. Auch hier zeigt sich, daß der funktionelle Zusammenhang zwischen Wobbeindex und Viskosität innerhalb vertretbarer Toleranzen praktisch unabhängig von der Temperatur ist. Eine gute Ermittlung der Viskosität, bei gleichzeitiger Berücksichtigung der Temperatur kann aber eine Steuerung eines Brenners bezüglich des ihm zugeführten Heizwertes über die dynamische Viskosität wesentlich verbessern.

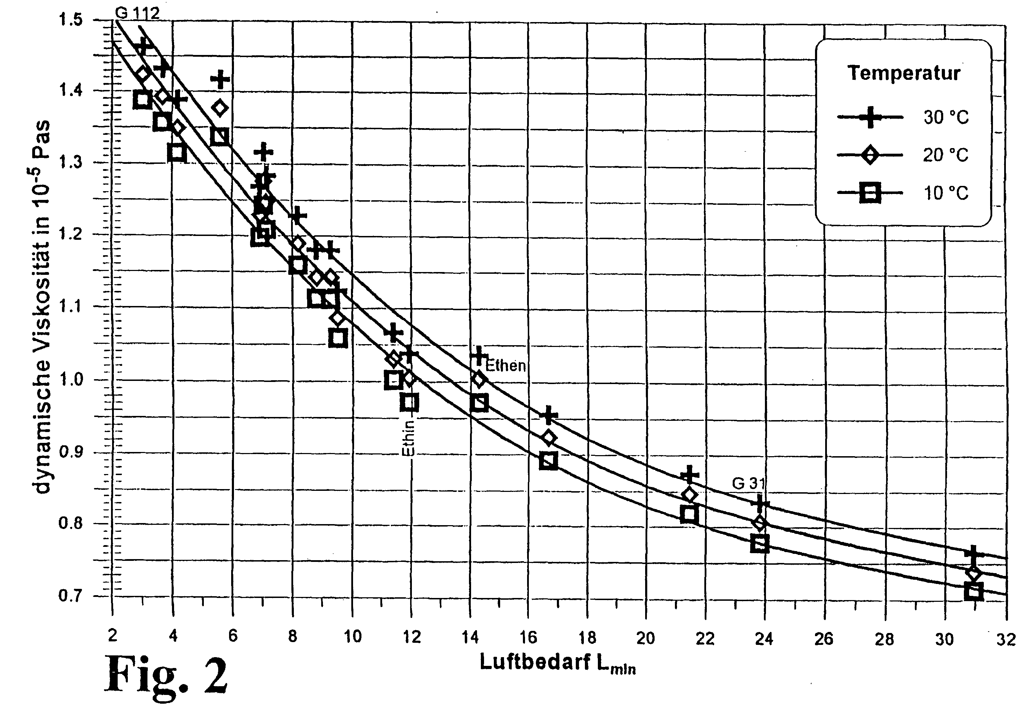

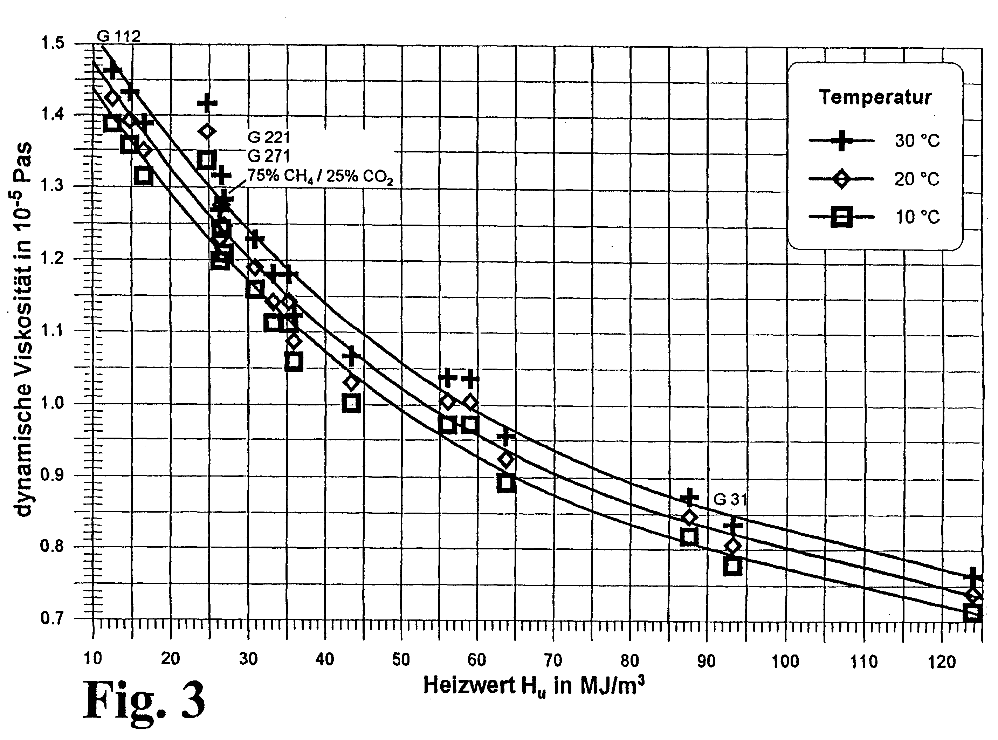

Mit Erhöhung des Heizwertes steigt auch der Oxidationsmittelbedarf zur vollständigen Verbrennung, der beispielsweise aus der Umgebungsluft zugeführt werden kann. Aufgrund der dargestellten Zusammenhänge sollte auch der Luftbedarf über eine Messung der dynamischen Viskosität steuerbar sein. Wie aus Fig. 2 ersichtlich ist, bei der für unterschiedliche Brennstoffe die dynamische Viskosität in Pascalsekunden gegenüber dem Luftbedarf aufgetragen ist, zeigt sich demgemäß ebenfalls ein einfacher Zusammenhang zwischen dem Luftbedarf und der dynamischen Viskosität mit nur geringen Abweichungen. Aus Fig. 2 wird deutlich, daß der geeignete Luftbedarf innerhalb der Grenzen von 10% aufgrund der gemessenen dynamischen Viskosität optimal eingestellt werden kann. Insbesondere gibt es auch einen ähnlichen Zusammenhang von dem Heizwert eines momentan aktuellen Brennstoffes mit der Viskosität, wie aus Fig. 3 zu ersehen ist.

Diese drei Figuren machen deutlich, daß eine Steuerung der Brenngaszusammensetzung unterschiedlichster Arten bezüglich des zugeführten Oxidationsmittels oder der Luft ideal geeignet für einen optimalen Brennerbetrieb ist.

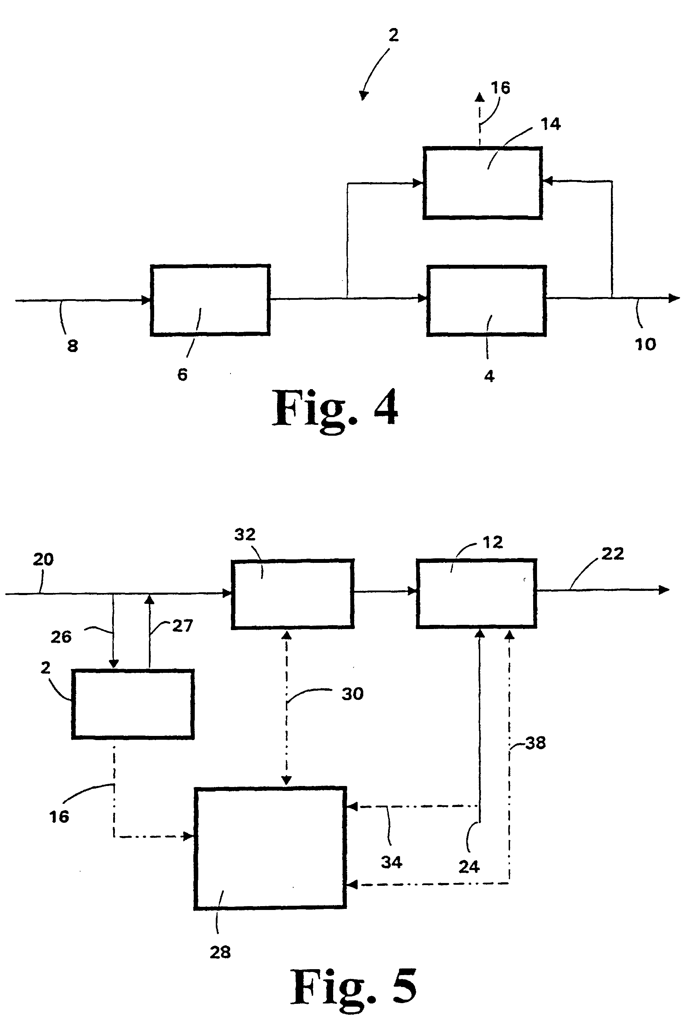

Ein besonders bevorzugtes Ausführungsbeispiel für einen Viskositätssensor 2 als Meßgerät für die Viskosität ist in Fig. 4 zu sehen. Ein derartiger Sensor ist auch als Rohrviskosimeter bekannt. Bei Auslegung dieses Viskositätssensors 2 wurde vor allen Dingen darauf geachtet, daß nur wenig Brennstoff zur Messung verwendet wird. Deswegen wurde als Rohr eine Kapillare 4, in die mit einer Mikropumpe 6 Brennstoff eingespeist wird, eingesetzt.

Die Zuführung des Brennstoffes erfolgte durch das Rohr 8, der Auslaß über das Rohr 10.

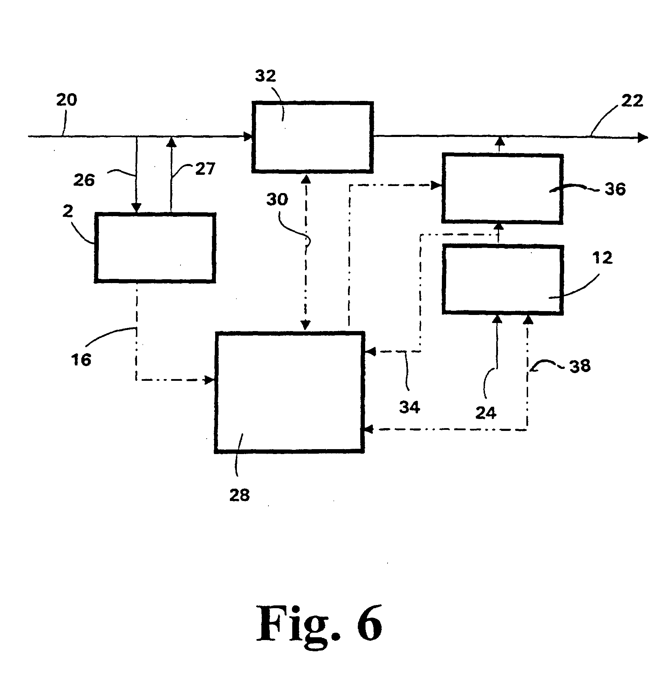

Man könnte den Brennstoff wegen der geförderten kleinen Mengen einfach in die Luft entweichen lassen. Wie jedoch in der Fig. 5 und Fig. 6 zu sehen ist, kann es auch dem Brennstoff wieder zugeführt werden.

Die Mikropumpe 6 in dem Ausführungsbeispiel von Figur 4 wurde für einen konstanten Volumenstrom angesteuert. Der Volumenstrom wurde dabei so gewählt, daß an der eingesetzten Kapillare 4 eine geeignet große Druckerhöhung stattfindet, um die Viskosität genau genug ermitteln zu können. Zur Ermittlung des Differenzdrucks am Auslauf und Einlauf der Kapillare 4 ist ein Drucksensor 14 vorgesehen. Der Drucksensor 14 ist ferner mit einem Temperatursensor ausgestattet, so daß beides, nämlich der Druckabfall sowie auch die Temperatur des Gases, für eine genauere Bestimmung der Viskosität zur Verfügung stehen.

Die durch den Druck- und Temperatursensor 14 erzeugten elektrischen Signale wurden einer Auswerteelektronik über eine Leitung 16 zugeführt. Die Auswerteelektronik bestand dabei im wesentlichen aus zwei Analog/Digital-Wandlern, mit denen die Signale für Druckdifferenz und Temperatur in digitale Werte gewandelt wurden. Die Auswerteelektronik enthielt weiter eine Prozessoreinheit mit der die digitalen Werte eingelesen werden konnten und welche einen Datenwert für die Viskosität aus den gewandelten Werten berechnete. Insbesondere wurde auch ein Programmablauf für den Prozessor vorgesehen, der den so ermittelten Datenwert für die Viskosität mit Hilfe einer Kennlinie in eine Steuergröße für das Zumischen von Luft oder Brennstoff zur Erzeugung des geeigneten Oxidationsmittel/Brennstoffgemisches wandelt.

Die dazu eingesetzte Kennlinie gestaltet sich besonders einfach, wenn die Strömung durch die Kapillare 4 im wesentlichen im laminaren Bereich liegt. Die Strömung könnte zwar auch im Übergangsbereichs zwischen laminar und turbulent liegen, ein Betrieb im Bereich turbulenter Strömung ist aber wegen der dann entstehenden Ungenauigkeit durch Ablösung von Wirbeln und Ähnlichem wenig zu empfehlen.

Deshalb wurde die Kapillare 4 für eine Reynoldszahl kleiner als 1000 ausgelegt. Insbesondere eignen sich für praktische Ausführungsbeispiele Rohrdurchmesser unterhalb von zwei Millimetern und insbesondere unterhalb von 0,2 Millimetern.

Insbesondere ergibt sich beim Ausführungsbeispiel Fig. 4, daß sowohl die Mikropumpe als mikromechanisches Bauelement als auch die Kapillare die Druckund Temperatursensoren auf einem einzigen Substrat verwirklicht werden können. Da derartige Bauelemente mit den in der Halbleitertechnik üblichen Verfahren hergestellt werden können, kann auch daran gedacht werden, sogar die Auswerteelektronik mit Analog/Digital-Wandlern und dem Prozessor auf demselben Substrat zu integrieren, so daß sich insgesamt ein besonders kleines Bauelement ergibt, das sich praktisch in jeder beliebigen Brennersteuerung einsetzen läßt.

Wie dieser Viskositätssensor 2 in einer Brennersteuerung integriert werden kann, ist vor allen Dingen in den Ausführungsbeispielen von Fig. 5 und Fig. 6 im einzelnen erläutert.

Fig. 5 und Fig. 6 unterscheiden sich im wesentlichen dadurch, daß in Fig. 5 die Brennstoffzufuhr geregelt wird, in Fig. 6 dagegen die Luftzufuhr.

Dabei sind zur Vereinfachung Bauelemente für die praktische Verwirklichung der Ausführungsform weggelassen worden. Bei dem entsprechendem Laboraufbau war beispielsweise vorgesehen, daß die Leitungern 8 und 10 mittels Ventilen von der Brennersteuerung abgekoppelt werden konnten und ein Referenzgas, beispielsweise die Umgebungsluft, zur Kalibrierung durch den Viskositätssensor geleitet wurde. Damit können auch mechanische Toleranzen, Änderungen im Drucksensor, der Pumpe u.ä. berücksichtigt werden, so daß auch über längere Zeiten ein ordnungsgemäßer Betrieb der Brennersteuerung auf unproblematische Weise möglich ist.

In beiden Ausführungsbeispielen von Fig. 5 und Fig. 6 wird der Brennstoff über eine Zuführungsleitung 20 eingeleitet und das über die Viskosität geeignet gemischte Oxidationsmittel/Brennstoffgemisch einem Brenner über eine Leitung 22 zugeleitet.

Das zur Verbrennung notwendige Oxidationsmittel wird dabei als Anteil der Umgebungsluft zugeführt, die über einen. Einlaß 24 in die dargestellten Vorrichtungen eintritt. Beiden Beispielen ist gemeinsam, daß der Brennstoff von der Zuführungsleitung 20 über eine Leitung 26 in den vorher beschriebenen Viskositätssensor 2 eingeleitet wird und die vermessene Brennstoffmenge anschließend dem Gesamtbrennstofffluß über die Leitung 27 wieder zugeführt wird. Die Stelle der Zufuhr ist für die Funktion eigentlich nicht kritisch. Es wurde jedoch festgestellt, daß die Messung und Einstellung besonders unkritisch sind, wenn die Leitung 27 nicht in die Brennstoffleitung 20 zurückgeführt wird, wie es in den Beispielen von Fig. 5 und Fig. 6 gezeigt ist, sondern in ein Gebläse 12, das zur Zufuhr der Luft vorgesehen ist, eingeleitet wird.

Die Meßwerte für die Gastemperatur, den Druck und den Volumenstrom werden über Zuleitungen 16 einer Steuereinrichtung 28 zugeführt, die nicht nur zur Implementierung der Zusatzfunktionen durch die Gasartenerkennung dient, sondern auch sämtlichen Aufgaben einer herkömmlichen Brennersteuerung erfüllt. Mit Hilfe einer Leitung 30 werden elektrische Signale an eine Gaseinheit 32 übermittelt, mit der die Brennstoffzufuhr in herkömmlicher Weise gesteuert wird. Weiter werden von der elektrischen Gaseinheit auch Daten, insbesondere manuelle Einstellungen für Sommer und Winterbetrieb, über die Leitung 30 an die Steuereinrichtung 28 übermittelt. Die Gaseinheit 32 kann sowohl elektrisch als auch pneumatisch ausgeführt sein. Im Falle eines pneumatischen Verbunds ist ein zusätzliches Stellglied notwendig.

Ferner ist eine weitere Leitung 34 in der Steuereinrichtung 28 gemäß den Figuren 5 und 6 gezeigt, die beispielsweise für eine Zusatzfunktion heranziehbar ist. In den Ausführungsbeispielen wird mit dieser Leitung 34 ein Signal für die Temperatur der Luft übertragen, um eine noch bessere Einstellung des Oxidationsmittel/Brennstoffgemisches zu ermöglichen. Natürlich können auch andere physikalische Werte der Umgebungsluft gemessen und zur Einstellung verwendet werden. Beispielsweise läßt sich mit einer derartigen Steuerungseinrichtung auch Luftdruck oder Luftfeuchtigkeit, wenn diese in Form geeigneter Signale zugeführt werden, über weitere Kennlinienscharen berücksichtigen.

Wie aus Fig. 5 zu erkennen ist, wird die Brennstoffmenge in diesem Beispiel durch die Gaseinheit 32 geregelt und die Luft bei einer Leistungseinstellung des Brenners praktisch konstant über das Gebläse 12 zugemischt, bevor das so entstehende Oxidationsmittel/Brennstoffgemisch in eine Zuführungsleitung 22 für den Brenner geleitet wird. Dagegen ist in Figur 6 hinter dem Gebläse 12 zum Zuführen der Luft ein Stellglied 36 vorgesehen, mit dem die Luftmenge geregelt wird. Zusätzlich wird auch das Gebläse 12 zur Bestimmung der Fördermenge von der Steuereinrichtung 28 über eine Steuerleitung 38 gesteuert.

Die vorher gezeigten Beispiele sind exemplarisch. Einleitend wurden schon mehrere Änderungsmöglichkeiten genannt. Insbesondere ist es möglich, den Druck am Viskositätssensor 2 konstant zu halten, indem die Mikropumpe 6 bezüglich einer konstanten Druckdifferenz über der Kapillare 4 angesteuert wird. Die Stellgröße zum Einstellen der Mikropumpe 6, insbesondere die Pumpfrequenz, ist iann direkt ein Maß für den Volumenstrom. Damit lassen sich Volumenstrom, Temperatur und Druck zur Steuerung einsetzten.

Insbesondere hat sich gezeigt, daß bei sehr einfachen Brennern, falls die Regelgenauigkeit nicht den höchsten Anforderungen entspricht, es keiner speziellen Temperaturmessung bedarf. Neben den in Beispiel Fig. 5 und Fig. 6 geregelten Gemischanteilen, Luft und Brennstoff, lassen sich auch andere Gase zumischen, um den Brenner gemäß dem Heizwert des zugeführten Brennstoffes zu steuern. Allen diesen Änderungsmöglichkeiten ist aber gemeinsam, daß die Viskosität über einen Viskositätssensor 2 so genau wie es für den jeweiligen Anwendungsfall erforderlich ist, ermittelt wird. Ein Signal für die dynamische Viskosität oder eine komplexe Funktion davon wird eingesetzt, um das Oxidationsmittel/Brennstoffgemisch in der Zuleitung 22 für einen Brenner zum optimalen Heizen beziehungsweise auch geringe Emission zu steuern.

Das hier vorgeschlagene Verfahren arbeitet völlig unabhängig von dem Brennerzustand. Dadurch läßt es sich auch schon in der Zündphase für den Brenner einsetzen, so daß auch in dieser Phase eine niedrigst mögliche Schadstoffemission verwirklicht werden kann und der Zündvorgang erleichtert wird.

Eine andere Möglichkeit des Einsatzes besteht darin, den Brenner entgegengesetzt der für Regelung über der für Ionisationsstrommessungen notwendigen Betriebsweise modulierend zu betreiben. Dies ist insbesondere bei einer Regelung der Brennstoff/Oxidationsmittel-Zusammensetzung mittels einer Ionisationsstrommessung mit technischen Schwierigkeiten verbunden, da sich der Referenzionisationsstrom mit der Leistungsänderung auch ändert.

Dieser kontinuierliche Betrieb reduziert die Heizkosten beträchtlich. Insbesondere besteht aber der Vorteil, daß aufgrund der vollständigeren Verbrennung im Vergleich zu vielen herkömmlichen Brennern auch die Wartungsintervalle verlängert werden, da der Brenner immer nur kurzzeitig auf Höchstleistung gefahren werden muß.

Insbesondere ist dabei hervorzuheben, daß auch die Abgasemission aufgrund einer optimalen Brennstoff/ Oxidationsmittelzusammensetzung wesentlich geringer ausfällt und daß insbesondere bei niederkalorischen Gasen durch die optimale Brennstoff/Oxidationsmittelzusammensetzung insgesamt ein höherer Brennwirkungsgrad erreicht wird, so daß auch die Heizkosten reduziert werden.

**Patentansprüche**

1. Vorrichtung zur Einstellung des Oxidationsmittel/Brennstoffgemisches in der Zuleitung (22) eines Brenners mit einer Einrichtung (32, 12) zur Änderung der Zusammensetzung des Oxidationsmittel/Brennstoffgemisches und einem Meßgerät (2) zur Erfassung des Zustandes des Oxidationsmittel/Brennstoffgemisches sowie einer Schaltung (28) zur Steuerung der Einrichtung (32, 12) zur Änderung der Zusammensetzung in Abhängigkeit des durch das Meßgerät erfaßten Zustands, **dadurch gekennzeichnet, daß** das Meßgerät den Heizwert bzw. den Wobbeindex von mindestens einem Teilgemisch des Oxidationsmittel/Brennstoffgemisches über die Viskosität oder eine Funktion derselben vor dem Verbrennen erfaßt und daß es in Strömungsrichtung vor der Einrichtung (32, 12) zur Änderung der Zusammensetzung angeordnet ist und die Zusammensetzung gemäß einer Funktion der erfaßten Viskosität mittels der Schaltung zur Steuerung der Einrichtung (32,12) steuert oder daß es hinter der Einrichtung (32, 12) zur Änderung der Zusammensetzung angeordnet ist und mittels der Schaltung zur Steuerung der Einrichtung (32, 12) die Zusammensetzung des Oxidationsmittel/Brennstoffgemisches regelt.

2. Vorrichtung nach Anspruch 1, **dadurch gekennzeichnet, daß** mindestens eine Meßstelle (14) zum Erfassen der Temperatur zumindest von einer Teilmenge des Oxidationsmittel/Brennstoffgemisches vorgesehen ist.

3. Vorrichtung nach Anspruch 1 oder 2, **dadurch gekennzeichnet, daß** das Meßgerät die Viskosität ausschließlich des Brennstoffs oder eine Funktion derselben erfaßt.

4. Vorrichtung nach einem der Ansprüche 1 bis 3, **dadurch gekennzeichnet, daß** das Meßgerät (2) ein Rohrviskosimeter ist, mit dem die dynamische Viskosität über den Zusammenhang zwischen Druckabfall über ein Rohr (4), den Volumenstrom und gegebenenfalls auch die Temperatur ermittelbar ist.

5. Vorrichtung nach Anspruch 4, **dadurch gekennzeichnet, daß** die Abmessungen des Rohrs (4) so gewählt sind, daß die Strömung im wesentlichen laminar ist, oder wenigstens im Übergangsbereich zwischen turbulenter und laminarer Strömung liegt.

6. Vorrichtung nach einem der Ansprüche 4 oder 5, **dadurch gekennzeichnet, daß** das Rohr (4) gerade ist.

7. Vorrichtung nach einem der Ansprüche 4 bis 6, **dadurch gekennzeichnet, daß** das Rohr einen gleichmäßigen, insbesondere kreisförmigen Querschnitt über seine Länge aufweist.

8. Vorrichtung nach einem der Ansprüche 4 bis 7, **dadurch gekennzeichnet, daß** der Rohrdurchmesser kleiner als 0,2 mm ist.

9. Vorrichtung nach einem der Ansprüche 4 bis 8, **dadurch gekennzeichnet, daß** der Druckabfall über das Rohr (4) oder zumindest in einem Teilrohrstück innerhalb des Rohres (4) messtechnisch erfaßbar ist.

10. Vorrichtung nach einem der Ansprüche 4 bis 9, **dadurch gekennzeichnet, daß** der Rohreinlauf gerundet ist.

11. Vorrichtung nach einem der Ansprüche 4 bis 10, **dadurch gekennzeichnet, daß** die Reynoldszahl des Rohres (4) kleiner als 1000 ist.

**12.** Vorrichtung nach einem der Ansprüche 4 bis 11, **dadurch gekennzeichnet, daß** eine steuerbare Pumpe (6) zur Einstellung des Volumenstroms in dem Rohr (4) vorgesehen ist.

**13.** Vorrichtung nach einem der Ansprüche 4 bis 12, **dadurch gekennzeichnet, daß** der Volumenstrom, insbesondere über eine steuerbare Pumpe (6), konstant geregelt ist.

**14.** Vorrichtung nach einem der Ansprüche 4 bis 12, **dadurch gekennzeichnet, daß** der Druckabfall über dem Rohr (4) mittels der Einstellung der Pumpe (6) geregelt ist.

**15.** Vorrichtung nach einem der Ansprüche 12 bis 14, **dadurch gekennzeichnet, daß** die Pumpe (6) eine Mikropumpe, insbesondere eine Halbleitermikropumpe, ist.

**16.** Vorrichtung nach Anspruch 15, **dadurch gekennzeichnet, daß** die Halbleitermikropumpe sowie mindestens Teile der Schaltung (28), insbesondere auch der Druck und Temperatursensor, auf einen gemeinsamen Substrat integriert sind.

**17.** Vorrichtung nach einem der Ansprüche 12 bis 16, **dadurch gekennzeichnet, daß** der Druckabfall über ein Rohr (4) über die Leistungsaufnahme der Pumpe bestimmbar ist.

**18.** Vorrichtung nach einem der Ansprüche 1 bis 17, **gekennzeichnet durch** eine Brennersteuerung (28), **durch** welche die Einstellung des Oxidationsmittels/Brennstoffgemisches schon vor der Zündung aktiv ist und gegebenenfalls die Zusammensetung des Oxidationsmittels/Brennstoffgemisches während des Brennens kontinuierlich steuerbar ist.

**19.** Vorrichtung nach einem der Ansprüche 1 bis 18, **dadurch gekennzeichnet, daß** eine Einrichtung (32, 12), insbesondere das Gebläse (12) des Brenners, zur Rückführung des Teilgemisches in die Zuleitung des Brenners vorgesehen ist, die den Ausgangsdruck des gemessenen Teilgemisches im wesentlichen unbeeinflußt läßt.

**20.** Vorrichtung nach einem der Ansprüche 1 bis 19, **dadurch gekennzeichnet, daß** sie ein Teil einer Gaseinheit (32) eines Brenners ist, indem sie in diesem Teil integriert ist.

**21.** Vorrichtung nach einem der Ansprüche 1 bis 20, **dadurch gekennzeichnet, daß** dem Meßgerät ein Referenzgas zuführbar ist.

**Claims**

**1.** Apparatus for adjusting the oxidizing agent/fuel mixture in the feed line (22) of a burner, having a device (32, 12) for changing the composition of the oxidizing agent/fuel mixture and a measurement appliance (2) for recording the state of the oxidizing agent/fuel mixture, as well as a circuit (28) for controlling the device (32, 12) for changing the composition as a function of the state recorded by the measurement appliance, **characterized in that** the measurement appliance records the calorific value or the Wobbe index of at least a partial mixture of the oxidizing agent/fuel mixture using the viscosity or a function thereof prior to combustion, and **in that** it is arranged upstream of the device (32, 12) for changing the composition, as seen in the direction of flow and provides open-loop control of the composition in accordance with a function of the recorded viscosity by means of the circuit for controlling the device (32, 12), or **in that** it is arranged downstream of the device (32, 12) for changing the composition and provides closed-loop control of the composition of the oxidizing agent/fuel mixture by means of the circuit for controlling the device (32, 12).

**2.** Apparatus according to Claim 1, **characterized in that** there is at least one measuring location (14) for recording the temperature of at least a partial quantity of the oxidizing agent/fuel mixture.

**3.** Apparatus according to Claim 1 or 2, **characterized in that** the measurement appliance records the viscosity only of the fuel or a function thereof.

**4.** Apparatus according to one of Claims 1 to 3, **characterized in that** the measurement appliance (2) is a tube viscometer which can be used to determine the dynamic viscosity by means of the relationship between pressure drop across a tube (4), the volumetric flow and if appropriate also the temperature.

5. Apparatus according to Claim 4, **characterized in that** the dimensions of the tube (4) are selected in such a way that the flow is substantially laminar or is at least in the transition range between turbulent and laminar flow.

6. Apparatus according to one of Claims 4 or 5, **characterized in that** the tube (4) is straight.

7. Apparatus according to one of Claims 4 to 6, **characterized in that** the tube has a uniform cross section, in particular a circular cross section, over its length.

8. Apparatus according to one of Claims 4 to 7, **characterized in that** the tube diameter is less than 0.2 mm.

9. Apparatus according to one of Claims 4 to 8, **characterized in that** the pressure drop across the tube (4) or at least in a partial section of tube inside the tube (4) can be recorded by metrological means.

10. Apparatus according to one of Claims 4 to 9, **characterized in that** the tube inlet is rounded.

11. Apparatus according to one of Claims 4 to 10, **characterized in that** the Reynolds number of the tube (4) is less than 1000.

12. Apparatus according to one of Claims 4 to 11, **characterized in that** there is a controllable pump (6) for setting the volumetric flow in the tube (4).

13. Apparatus according to one of Claims 4 to 12, **characterized in that** the volumetric flow is controlled so as to be constant, in particular by means of a controllable pump (6).

14. Apparatus according to one of Claims 4 to 12, **characterized in that** the pressure drop across the tube (4) is controlled by setting the pump (6).

15. Apparatus according to one of Claims 12 to 14, **characterized in that** the pump (6) is a micropump, in particular a semiconductor micropump.

16. Apparatus according to Claim 15, **characterized in that** the semiconductor micropump and at least parts of the circuit (28), in particular also the pressure and temperature sensor, are integrated on a common substrate.

17. Apparatus according to one of Claims 12 to 16, **characterized in that** the pressure drop across a tube (4) can be determined by means of the power consumption of the pump.

18. Apparatus according to one of Claims 1 to 17, **characterized by** a burner control (28), by means of which the setting of the oxidizing agent/fuel mixture is active even before ignition and if appropriate the composition of the oxidizing agent/fuel mixture can be controlled continuously during combustion.

19. Apparatus according to one of Claims 1 to 18, **characterized in that** there is a device (32, 12), in particular the blower (12) of the burner, for returning the partial mixture into the burner feed line, leaving the exit pressure of the measured partial mixture substantially unaffected.

20. Apparatus according to one of Claims 1 to 19, **characterized in that** it is part of a gas unit (32) of a burner by virtue of being integrated in this part.

21. Apparatus according to one of Claims 1 to 20, **characterized in that** a reference gas can be fed to the measurement appliance.

## Revendications

1. Dispositif pour le réglage du mélange oxydant / combustible dans la conduite d'alimentation (22) d'un brûleur, comportant un dispositif (32, 12) servant à la modification de la composition du mélange oxydant / combustible, et un appareil de mesure (2) prévu pour la détermination de l'état du mélange oxydant / combustible, ainsi qu'un circuit (28) servant à la commande du dispositif (32, 12) pour la modification de la composition en fonction de l'état déterminé par l'appareil de mesure, **caractérisé en ce que** l'appareil de mesure détermine la valeur calorifique ou bien l'indice de Wobbe d'au moins un mélange partiel du mélange oxydant / combustible, par l'intermédiaire de la viscosité ou d'une fonction de celle-ci avant la combustion, et **en ce que** l'appareil de mesure est disposé, dans le sens d'écoulement, avant le dispositif (32, 12) pour la modification de la composition et commande la composition selon une fonction de la viscosité détectée, à l'aide du circuit servant à la commande du dispositif (32, 12), ou **en ce que** l'appareil de mesure est disposé après le dispositif (32, 12) pour la modification de la composition et règle la composition du mélange oxydant / combustible, à l'aide du circuit servant à la commande du dispositif (32, 12).

**2.** Dispositif selon la revendication 1, **caractérisé en ce qu'**il est prévu au moins un emplacement de mesure (14) pour déterminer la température au moins d'une quantité partielle du mélange oxydant / combustible.

**3.** Dispositif selon la revendication 1 ou 2, **caractérisé en ce que** l'appareil de mesure détermine la viscosité exclusivement du combustible, ou bien une fonction de cette viscosité.

**4.** Dispositif selon l'une des revendications 1 à 3, **caractérisé en ce que** l'appareil de mesure (2) est un viscosimètre tubulaire, qui permet de déterminer la viscosité dynamique par l'intermédiaire du rapport existant entre la chute de pression à travers un tube (4), le flux volumique et, le cas échéant, également la température.

**5.** Dispositif selon la revendication 4, **caractérisé en ce que** les dimensions du tube (4) sont choisies de telle sorte que l'écoulement soit essentiellement laminaire, ou bien qu'il soit au moins situé dans la zone de transition entre écoulement turbulent et écoulement laminaire.

**6.** Dispositif selon la revendication 4 ou 5, **caractérisé en ce que** le tube (4) est rectiligne.

**7.** Dispositif selon l'une des revendications 4 à 6, **caractérisé en ce que** le tube présente une section transversale régulière, notamment circulaire, sur toute sa longueur.

**8.** Dispositif selon l'une des revendications 4 à 7, **caractérisé en ce que** le diamètre du tube est inférieur à 0,2 mm.

**9.** Dispositif selon l'une des revendications 4 à 8, **caractérisé en ce que** la chute de pression à travers le tube (4) ou au moins dans un tronçon tubulaire à l'intérieur du tube (4) peut être déterminée par une technique de mesure.

**10.** Dispositif selon l'une des revendications 4 à 9, **caractérisé en ce que** l'entrée du tube est arrondie.

**11.** Dispositif selon l'une des revendications 4 à 10, **caractérisé en ce que** le nombre de Reynolds du tube (4) est inférieur à 1000.

**12.** Dispositif selon l'une des revendications 4 à 11, **caractérisé en ce qu'**il est prévu une pompe (6), pouvant être commandée, pour le réglage de l'écoulement volumique dans le tube (4).

**13.** Dispositif selon l'une des revendications 4 à 12, **caractérisé en ce que** l'écoulement volumique est réglé pour être constant, notamment par l'intermédiaire d'une pompe (6) pouvant être commandée.

**14.** Dispositif selon l'une des revendications 4 à 12, **caractérisé en ce que** la chute de pression à travers le tube (4) est réglée à l'aide du réglage de la pompe (6).

**15.** Dispositif selon l'une des revendications 12 à 14, **caractérisé en ce que** la pompe (6) est une micropompe, notamment une micropompe à semi-conducteurs.

**16.** Dispositif selon la revendication 15, **caractérisé en ce que** la micropompe à semi-conducteurs, ainsi qu'au moins des éléments du circuit (28), et notamment aussi le capteur de pression et le capteur de température, sont intégrés sur un substrat commun.

**17.** Dispositif selon l'une des revendications 12 à 16, **caractérisé en ce que** la chute de pression à travers un tube (4) peut être déterminée par l'intermédiaire de la puissance absorbée par la pompe.

**18.** Dispositif selon l'une des revendications 1 à 17, **caractérisé par** un dispositif de commande de brûleur (28), au moyen duquel le réglage du mélange oxydant / combustible est déjà mis en fonction avant l'allumage et, le cas échéant, la composition du mélange oxydant / combustible peut être commandée en continu pendant la combustion.

19. Dispositif selon l'une des revendications 1 à 18, **caractérisé en ce qu'**il est prévu un dispositif (32, 12), notamment la soufflante (12) du brûleur, pour réinjecter le mélange partiel dans la conduite d'alimentation du brûleur, qui laisse sensiblement non influencée la pression de départ du mélange partiel mesuré.

20. Dispositif selon l'une des revendications 1 à 19, **caractérisé en ce qu'**il constitue une partie d'une unité de gaz (32) d'un brûleur, en étant intégré dans cette partie.

21. Dispositif selon l'une des revendications 1 à 20, **caractérisé en ce qu'**un gaz de référence peut être amené à l'appareil de mesure.

**Fig. 1**

Fig. 2

EP 1 173 710 B1

**Fig. 3**

EP 1 173 710 B1

# Fig. 4

# Fig. 5

Fig. 6